# FASCICULE DE BREVET EUROPEEN

(11) **EP 0 577 449 B1**
(45) Date de publication et mention de la délivrance du brevet: **13.03.1996**
(21) Numéro de dépôt: 93401441.6
(22) Date de dépôt: 07.06.1993
(51) Int. Cl.: C07C 219/06, D06M 15/423

(54) **Tris (acétoacétoxy-2 éthyl)- amine, et ses sels hydrosolubles, procédé de préparation, application à titre de capteurs de formaldéhyde et procédé d'ennoblissement de tissus**
Tris-(acetoacetoxy-2-ethyl)-Amin und ihren wasserlöslichen Salzen, Verfahren zu ihrer Herstellung, Verwendung als Formaldehyd-Fänger und Verfahren zur Veredlung von Textilien
Tris-(acetoacetoxy-2-ethyl)-amine and its water soluble salts, process for their preparation, use as formaldehyde scavengers and process for the finishing of textiles

(30) Priorité: 25.06.1992 FR 9207798
(43) Date de publication de la demande: 05.01.1994
(73) Titulaire: SOCIETE FRANCAISE HOECHST Société anonyme dite:, F-92800 Puteaux (FR)
(72) Inventeur: Wilhelm, Didier, F-92130 Issy les Moulineaux (FR); Gelabert, Antonio, F-95570 Bouffemont-Moiselles (FR)
(74) Mandataire: Rinuy, Santarelli

(56) Documents cités:
- EP-A- 0 378 067
- WO-A-88/09323
- DATABASE WPIL Section Ch, Week 7323, Derwent Publications Ltd., London, GB; Class A, AN 73-33350U & JP-B-48 017 755
- TEXTILE CHEMISTS AND COLORISTS vol. 16, no. 12, 1984, RESEARCH TRIANGLE PARK, N.C. pages 33 - 38 C. TOMASINO ET AL. 'EVALUATION OF FORMALDHYDE SCVENGERS'

## Description

La présente invention concerne la tris (acétoacétoxy-2 éthyl)-amine, ses sels d'acide minéral ou organique hydrosolubles, leur procédé de préparation, leur application à titre de capteurs de formaldéhyde et un procédé d'ennoblissement de tissus.

Le formaldéhyde est largement utilisé dans la préparation de résines aminoplastes diverses, notamment pour l'obtention de résines destinées à l'ennoblissement des fibres textiles telles que les résines urées-glyoxal-formaldéhyde. Malgré le soin apporté à leur préparation, ces résines contiennent très souvent de faibles traces de formaldéhyde et, sous certaines conditions, notamment lors de leur application sur les tissus, elles libèrent du formaldéhyde. Or, le formaldéhyde est un gaz d'odeur piquante, très pénétrante, peu agréable ; aussi a-t-on cherché très tôt divers moyens pour le fixer et/ou limiter son dégagement. Ainsi, comme capteur de formaldéhyde, il a été proposé divers dérivés carbonylés possédant en position alpha un groupe méthyle ou méthylène tels que l'acétaldéhyde, l'acétone, l'acétylacétone, des esters éther de l'acide acétylacétique et même plus généralement des composés à méthylène actif comme les nitroalcanes, les malonates de dialcanol (brevet français N° 989465 et 2575754, demande de brevet européen N° 2596, brevet anglais N° 2058099, brevet polonais N° 72885, demande de brevet japonais N° 73-17755, demande de brevet PCT N° WO88/09323 et C.TOMASINO et al, Textile Chemists and Colorists, 1984, 16, (12), 33-38).

Toutefois, ces divers produits quoique actifs, ne donnent pas entière satisfaction à l'homme du métier.

Or, la demanderesse a découvert avec étonnement que la tris (acétoacétoxy-2 éthyl)-amine, et ses sels d'acide minéral ou organique, hydrosolubles présentent d'excellentes propriétés pour capter le formaldéhyde présent dans les tissus à base de fibres cellulosiques ennoblis avec des résines aminoplastes contenant à l'état libre ou combiné du formaldéhyde.

C'est pourquoi la présente invention a pour objet la tris (acétoacétoxy-2 éthyl)-amine de formule (I)

N (CH₂-CH₂-O-CO-CH₂-CO-CH₃)₃ (I)

ainsi que ses sels avec les acides minéraux ou organiques hydrosolubles.

Les sels d'acides minéraux et organiques de la tris(acétoacétoxy-2 éthyl)-amine solubles dans l'eau, sont les sels d'acides usuels. Parmi les acides usuels, on peut citer l'acide chlorhydrique, l'acide nitrique, l'acide sulfurique, l'acide orthophosphorique, l'acide phosphonique, l'acide phosphinique, l'acide paratoluènesulfonique, l'acide succinique, l'acide maléique, l'acide benzoïque, l'acide citrique.

Selon l'invention la tris(acétoacétoxy-2 éthyl)-amine et ses sels peuvent être préparés par un procédé caractérisé en ce que l'on fait réagir le triméthyl-2, 2, 6-oxo-4-dioxine-1, 3 avec la triéthanolamine, pour obtenir la tris(acétoacétoxy-2 éthyl)-amine que, si désiré, l'on salifie selon les méthodes usuelles.

Dans des conditions préférentielles de mise en oeuvre de l'invention, la condensation du triméthyl-2, 2, 6-oxo-4 dioxine-1,3 avec la triéthanolamine est réalisée :
- à une température comprise entre 55 et 150°C,
- avec élimination par distillation de l'acétone formée,
- en présence d'un solvant possédant un point d'ébullition supérieur à celui de l'acétone tel qu'un hydrocarbure aromatique (benzène, toluène, xylène).

Comme on l'a dit précédemment, la tris(acétoacétoxy-2 éthyl)-amine, et ses sels d'acide minéral ou organique hydrosolubles présentent de très intéressantes propriétés comme capteurs de formaldéhyde, et notamment, ils permettent d'abaisser très considérablement les doses de formaldéhyde libre présent et/ou libéré par les tissus à base de fibres cellulosiques, apprêtés avec des résines aminoplastes contenant à l'état libre ou combiné du formaldéhyde.

Dans son application dans le domaine de l'ennoblissement des tissus à base de fibres cellulosiques avec des résines aminoplastes couramment utilisées pour conférer aux tissus des propriétés d'infroissabilité telles que les résines classiques urée-glyoxal-formaldéhyde, éthérifiées ou non avec un alcanol tel que le méthanol, on utilise la tris(acétoacétoxy-2 éthyl)-amine, ou l'un de ses sels d'acide minéral ou organique hydrosolubles en solution dans le bain d'apprêtage de préférence à la dose de 0,001 à 2 moles par litre de bain d'apprêt et l'on effectue ensuite l'apprêtage dans les conditions habituelles. Après traitement, les tissus ainsi apprêtés ne contiennent pratiquement plus de formaldéhyde libre et /ou susceptible de se libérer.

C'est pourquoi la présente demande a aussi pour objet un procédé d'ennoblissement des tissus à base de fibres cellulosiques au moyen d'une résine aminoplaste contenant à l'état libre ou combiné du formaldéhyde dans lequel on place les tissus dans un bain d'ennoblissement, caractérisé en ce que l'on utilise dans ledit bain d'ennoblissement, à titre de capteur de formaldéhyde, la tris(acétoacétoxy-2 éthyl)-amine, ou l'un de ses sels d'acide minéral ou organique hydrosolubles.

Les exemples suivants sont donnés à titre indicatif ; ils permettent de mieux comprendre l'invention.

Sauf indications contraires, les parties et pourcentages sont donnés en poids. Le test de défroissabilité est réalisé selon la norme AATCC 66-1972, la défroissabilité est exprimée par la somme des angles de défroissement obtenus en sens chaîne et en sens trame. La résistance à la traction des échantillons, exprimée en daN, en sens chaîne plus en sens trame est réalisée selon la norme AFNOR G 07.001. Le jaunissement des tissus, réalisé sur un appareil FIXOTEST à 185°C pendant 30 secondes et les blancheurs, exprimées en degré Berger, sont mesurées avec un spectrophotomètre.

Les taux de formaldéhyde, exprimés en ppm, sont déterminés selon la norme AATCC 112-1984, désignée ci-après AATCC 112.

### EXEMPLE 1

On chauffe deux heures à 120°C avec distillation concomitante de l'acétone formée :
- 251 g de triméthyl 2,2,6 oxo-4 dioxine-1,3 à 85%, soit 1,5 mole,
- 74,6 g (0,5 mole) de triéthanolamine,
- 265 g d'o-xylène,
puis on élimine sous pression réduite environ 220 g d'oxylène. Le milieu réactionnel refroidi est ensuite repris avec 400 g d'eau distillée. On décante et la phase organique est relavée deux fois avec 400 g d'eau distillée. Les phases aqueuses d'extraction sont réunies, puis elles sont lavées avec 30 g de pentane, et enfin, elles sont concentrées sous pression réduite. L'huile résiduelle est reprise avec 530 g de dichlorométhane, puis le mélange obtenu est séché sur sulfate de magnésium anhydre, filtré et concentré sous pression réduite. On obtient ainsi 144,1 g d'une huile orangée dont le spectre RMN du ¹³C, pris en solution dans le diméthylsulfoxyde deutéré est conforme à la tris(acétoacétoxy-2 éthyl)-amine : RMN ¹³C à 50 MHz (DMSO) δppm/TMS : 30 (CH₃), 49,6 (N-CH₂), 52,6 (CH₂) , 63 (CH₂-O), 167,3 (COO), 201,5 (CO) ; RMN ¹H à 200 MHz (DMSOd₆) δppm/TMS : 2,17 (s, 9H, CH₃), 2,76 (t, 6H, d=5,7 Hz, N-CH₂), 3,54 (s, 6H, CH₂), 4,06 (t, 6H, J=5,7 Hz, O-CH₂).

| Microanalyse pour C₁₈ H₂₇ NO₉ : 401,41 | | | | | | | | |
|---|---|---|---|---|---|---|---|---|
| Calculé | C = | 53,86 | H = | 6,78 | N = | 3,49 | O = | 35,87 |
| Trouvé | | 53,4 | | 6,9 | | 3,5 | | |

### EXEMPLE 2

On imprègne au foulard un tissu de popeline 100% coton, débouilli et blanchi, d'un poids d'environ 130 g par mètre carré avec un taux d'exprimage de 75 % dans un bain aqueux, dont le pH est indiqué dans le tableau I, contenant en solution :
- 55 g/l d'une résine commerciale vendue par la demanderesse sous la désignation ARKOFIX^{R} NFL Conc. qui est une résine urée-glyoxal-formaldéhyde faiblement éthérifiée au méthanol,
- 15 g/l d'un catalyseur commercialisé par la demanderesse sous la désignation catalyseur 3282,
- 2 g/l d'un agent mouillant : le nonylphénol éthoxylé avec 10 moles d'oxyde d'éthylène,
- 100 mmoles/l de tris(acétoacétoxy-2 éthyl)-amine préparé à l'exemple 1.

Le tissu est ensuite séché pendant 45 secondes à 120°C, puis il est soumis sur une rame de laboratoire à un traitement thermique de 35 secondes à 170°C.

On détermine ensuite sur des échantillons du tissu traité :
- la défroissabilité,
- la résistance à la traction, désignée ci-après Rt,
- la blancheur, désignée ci-après B,
- le jaunissement, désigné ci-après J,
- le taux de formaldéhyde libre, exprimé en ppm, désigné ci-après F
- la résistance au chlore.

Les résultats obtenus sont donnés dans le tableau I en comparaison avec un tissu non traité (exemple 3) et un tissu traité de manière conventionnelle en l'absence d'un capteur de formaldéhyde (exemple 4).

L'examen du tableau I permet de constater que les tissus apprêtés en présence de tris(acétoacétoxy-2 éthyl)-amine selon la présente invention ne contiennent pratiquement plus de formaldéhyde.

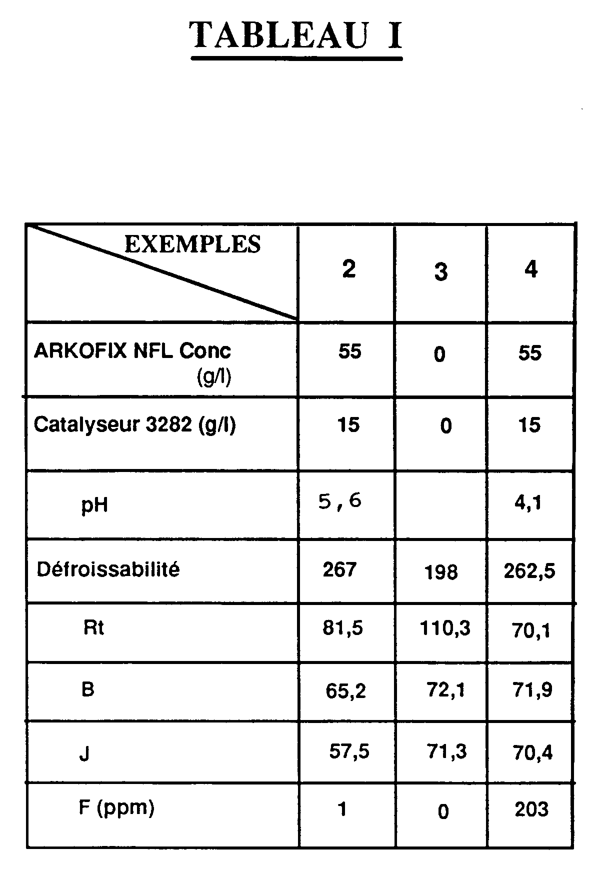

## Revendications

1. Tris(acétoacétoxy-2 éthyl)-amine ainsi que ses sels d'acide minéral ou organique hydrosolubles.

2. Procédé de préparation de la tris(acétoacétoxy-2 éthyl)-amine, ainsi que de ses sels d'acides minéraux ou organiques caractérisé en ce que l'on fait réagir le triméthyl-2, 2, 6-oxo-dioxine-1, 3 avec la triéthanolamine pour obtenir la tris(acétoacétoxy-2 éthyl)-amine que, si désiré, l'on salifie selon les méthodes usuelles.

3. Utilisation de la tris(acétoacétoxy-2 éthyl)-amine, ainsi que de ses sels d'acides minéraux ou organiques hydrosolubles à titre de capteurs de formaldéhyde.

4. Procédé d'ennoblissement des tissus à base de fibres cellulosiques au moyen d'une résine aminoplaste contenant à l'état libre ou combiné du formaldéhyde dans lequel on place les tissus dans un bain d'ennoblissement, caractérisé en ce que l'on utilise dans ledit bain d'ennoblissement, à titre de capteur de formaldéhyde, la tris(acétoacétoxy-2 éthyl)-amine, ou l'un de ses sels d'acide minéral ou organique hydrosolubles.

5. Procédé selon la revendication 4, caractérisé en ce que l'on utilise la tris(acétoacétoxy-2 éthyl)-amine, ou de l'un de ses sels d'acide minéral ou organique hydrosolubles en solution à la dose de 0,001 à 2 moles par litre de bain d'ennoblissement,

## Claims

1. Tris(2-acetoacetoxy ethyl)-amine as well as its hydrosoluble mineral or organic acid salts.

2. Preparation process for tris(2-acetoacetoxy ethyl)-amine, as well as its mineral or organic acid salts, characterized in that 2,2,6-trimethyl-oxo-1,3-dioxine is reacted with triethanolamine in order to obtain tris(2-acetoacetoxy ethyl)-amine which, if desired, is salified according to the usual methods.

3. Use of tris(2-acetoacetoxy ethyl)-amine, as well as its hydrosoluble mineral or organic acid salts, as formaldehyde collectors.

4. Finishing process for fabrics based on cellulose fibres using an aminoplastic resin containing formaldehyde in the free or combined state in which the fabrics are placed in a finishing bath, characterized in that tris(2-acetoacetoxy ethyl)-amine, or one of its hydrosoluble mineral or organic acid salts, is used as the formaldehyde collector in the said finishing bath.

5. Process according to claim 4, characterized in that tris(2-acetoacetoxy ethyl)-amine, or one of its hydrosoluble mineral or organic acid salts, is used in solution at a dose of 0.001 to 2 moles per litre of finishing bath.

## Patentansprüche

1. Tris-(acetoacetoxy-2-ethyl)-amin und seine wasserlöslichen Salze von Mineralsäuren oder organischen Säuren.

2. Verfahren zur Herstellung von Tris-(acetoacetoxy-2-ethyl)-amin und seinen Salzen von Mineralsäuren oder organischen Säuren, dadurch gekennzeichnet, daß man 2,2,6-Trimethyl-4-oxo-1,3-dioxan mit Triethanolamin zur Erzielung von Tris-(acetoacetoxy-2-ethyl)-amin umsetzt, das gewünschtenfalls nach herkömmlichen Verfahren in ein Salz umgewandelt wird.

3. Verwendung von Tris-(acetoacetoxy-2-ethyl)-amin und seiner wasserlöslichen Salze von Mineralsäuren oder organischen Säuren als Formaldehydfänger.

4. Veredelungsverfahren für Gewebe auf der Basis von Cellulosefasern mittels eines Aminoplastharzes, das Formaldehyd in freiem oder gebundenem Zustand enthält, bei welchem die Gewebe in ein Veredelungsbad gegeben werden, dadurch gekennzeichnet, daß man in diesem Verdelungsbad als Formaldehydfänger Tris-(acetoacetoxy-2-ethyl)-amin oder eines seiner wasserlöslichen Salze von Mineralsäuren oder organischen Säuren verwendet.

5. Verfahren nach Anspruch 4, dadurch gekennzeichnet, daß man das Tris-(acetoacetoxy-2-ethyl)-amin oder eines seiner wasserlöslichen Salze von Mineralsäuren oder organischen Säuren in Lösung in einer Menge von 0,001 bis 2 Mol/l Veredelungsbad verwendet.
